# EUROPÄISCHE PATENTANMELDUNG

(11) **EP 3 514 223 A1**
(43) Veröffentlichungstag der Anmeldung: **24.07.2019**
(21) Anmeldenummer: 18152153.5
(22) Anmeldetag: 17.01.2018
(51) Int. Cl.: C12M 1/00, C12M 1/21, C12M 1/34

(54) **MULTISENSOR FÜR EINEN BIOREAKTOR, BIOREAKTOR, VERFAHREN ZUR HERSTELLUNG EINES MULTISENSORS UND ZUR MESSUNG VON PARAMETERN**

(71) Anmelder: Eppendorf AG, 22339 Hamburg (DE)
(72) Erfinder: Selzer, Sebastian, 52074 Aachen (DE); Petkau, Rudolf, 52062 Aachen (DE); Ertel, Guido, 41539 Dormagen (DE); Streule, Wolfgang, 52445 Titz-Rödingen (DE)
(74) Vertreter: Eisenführ Speiser

(57) **Zusammenfassung**

Die Erfindung betrifft einen Multisensor für einen Bioreaktor zur Anwendung in der Zellkultur und/oder der Mikrobiologie, einen Bioreaktor zur Anwendung in der Zellkultur und/oder der Mikrobiologie, ein Verfahren zur Herstellung eines Multisensors und ein Verfahren zur Messung von Parametern in einem Bioreaktor zur Anwendung in der Zellkultur und/oder der Mikrobiologie. Der Multisensor umfasst mindestens drei Messanordnungen, die ausgebildet sind, mindestens drei Parameter zu messen, wobei eine erste der drei Messanordnungen ausgebildet ist, eine Impedanzmessung und/oder eine kapazitive Messung durchzuführen, und wobei die erste Messanordnung mindestens zwei Elektroden aufweist, die einen elektrisch leitfähigen Kunststoff umfassen oder daraus bestehen.

## Beschreibung

Die Erfindung betrifft einen Multisensor für einen Bioreaktor zur Anwendung in der Zellkultur und/oder der Mikrobiologie, einen Bioreaktor zur Anwendung in der Zellkultur und/oder der Mikrobiologie, ein Verfahren zur Herstellung eines Multisensors und ein Verfahren zur Messung von Parametern in einem Bioreaktor zur Anwendung in der Zellkultur und/oder der Mikrobiologie.

Bioreaktoren, Sensoren und Verfahren zur Messung von Parametern sind bekannt beispielsweise aus EP 2725095 B1, WO 2016092281 A1, CN 105044038 A. Insbesondere für die Anwendung in der Zellkultur und/oder der Mikrobiologie sind jedoch weitere Verbesserungen wünschenswert.

Es ist daher eine Aufgabe der vorliegenden Erfindung, einen verbesserten Multisensor für einen Bioreaktor zur Anwendung in der Zellkultur und/oder der Mikrobiologie, einen verbesserten Bioreaktor zur Anwendung in der Zellkultur und/oder der Mikrobiologie, ein verbessertes Verfahren zur Herstellung eines Multisensors und ein verbessertes Verfahren zur Messung von Parametern in einem Bioreaktor zur Anwendung in der Zellkultur und/oder der Mikrobiologie bereitzustellen.

Insbesondere ist es eine Aufgabe der vorliegenden Erfindung, einen Bioreaktor zur Anwendung in der Zellkultur und/oder der Mikrobiologie, ein Verfahren zur Herstellung eines Multisensors und ein Verfahren zur Messung von Parametern in einem Bioreaktor zur Anwendung in der Zellkultur und/oder der Mikrobiologie bereitzustellen, welche kostengünstig sind. Es ist insbesondere auch eine Aufgabe der vorliegenden Erfindung, einen Bioreaktor zur Anwendung in der Zellkultur und/oder der Mikrobiologie, ein Verfahren zur Herstellung eines Multisensors und ein Verfahren zur Messung von Parametern in einem Bioreaktor zur Anwendung in der Zellkultur und/oder der Mikrobiologie bereitzustellen, welche auch für kleine Arbeitsvolumina geeignet sind.

Diese Aufgabe wird erfindungsgemäß gelöst durch einen Multisensor für einen Bioreaktor zur Anwendung in der Zellkultur und/oder der Mikrobiologie, der Multisensor umfasst mindestens drei Messanordnungen, die ausgebildet sind, mindestens drei Parameter zu messen, wobei eine erste der drei Messanordnungen ausgebildet ist, eine Impedanzmessung und/oder eine kapazitive Messung durchzuführen, und wobei die erste Messanordnung mindestens zwei Elektroden aufweist, die einen elektrisch leitfähigen Kunststoff umfassen oder daraus bestehen.

Der hier beschriebene Multisensor ist somit ausgebildet, mittels der mindestens drei Messanordnungen mindestens drei Parameter zu messen, wobei vorzugsweise mindestens drei unterschiedliche Messanordnungen zum Einsatz kommen und/oder mindestens drei unterschiedliche Parameter gemessen werden können. Die erste der drei Messanordnungen ist geeignet, eine Impedanzmessung und/oder eine kapazitive Messung durchzuführen. Hierzu weist die erste Messanordnung, zwei oder mehrere Elektroden auf, die einen elektrisch leitfähigen Kunststoff umfassen oder daraus bestehen.

Die Erfindung beruht unter anderem auf der Erkenntnis, dass gerade für die Anwendung in der Zellkultur und/oder der Mikrobiologie häufig nur wenig Platz zur Anordnung von Sensoren an und/oder in Bioreaktoren besteht. Gleichzeitig bestehen oft hohe Anforderungen hinsichtlich möglichst niedriger Kosten. Mit dem hier beschriebenen Multisensor können mindestens drei Parameter gemessen werden, so dass gegenüber Einzelsensoren mit dem Multisensor die Plätze von zwei Sensoren eingespart werden können. Dabei ist die erste Messanordnung des Multisensors für eine Impedanzmessung, insbesondere mittels Impedanzspektroskopie und/oder dielektrischer Spektroskopie, und/oder eine kapazitive Messung ausgebildet, was besonders bevorzugt und in vielen Anwendungsbereichen wichtige Messverfahren sind. Dadurch, dass mindestens zwei Elektroden der ersten Messanordnung einen elektrisch leitfähigen Kunststoff umfassen oder daraus bestehen, kann eine besonders kostengünstige Ausbildung des Multisensors realisiert werden.

Bioreaktoren, die häufig auch als Fermenter bezeichnet werden, schließen in der Regel einen Reaktionsraum ein, in dem biologische bzw. biotechnologische Prozesse im Labormaßstab durchgeführt werden können. Zu solchen Prozessen zählt beispielsweise die Kultivierung von Zellen, Mikroorganismen oder kleinen Pflanzen unter definierten, vorzugsweise optimierten, kontrollierten und reproduzierbaren Bedingungen. Bioreaktoren verfügen dazu meist über mehrere Anschlüsse, über die Primär- und Sekundärstoffe sowie verschiedene Instrumente, wie beispielsweise Sensoren, in den Reaktionsraum eingebracht werden können oder über die beispielsweise Fluidleitungen, insbesondere Gasleitungen, wie Begasungs- oder Abgasleitungen, angeschlossen werden können. Bioreaktoren weisen ferner in der Regel ein Rührwerk auf, dessen Rührwelle von einem Antrieb in Rotation versetzt werden kann, wodurch meist ein drehsteif mit der Rührwelle verbundenes Rührelement ebenfalls in Rotation versetzt wird und so eine Durchmischung der im Reaktionsraum vorhandenen Stoffe bewirkt. Es können auch zwei oder mehr Rührelemente, meist axial beabstandet, auf der Rührwelle angeordnet und mit dieser verbunden sein. Das Rührelement bzw. die Rührelemente kann bzw. können auch einstückig mit der Rührwelle ausgebildet sein. Bioreaktoren können verschiedene Geometrien aufweisen. Formstabile Bioreaktoren können beispielsweise einen Querschnitt aufweisen, vorzugsweise einen Querschnitt in einer im Betriebszustand horizontalen Ebene, der im Wesentlichen kreisförmig, oval, dreieckig, rechteckig, quadratisch, trapezförmig, polygonal oder eine Freiform ist. Flexible Bioreaktoren können beispielsweise als Beutel ausgebildet sein und können optional formstabile Anschlusseinrichtungen aufweisen.

Sowohl für den Anwendungsbereich der Zellkultivierung als auch für den mikrobiologischen Anwendungsbereich ist die Verwendung von Bioreaktoren in, vorzugsweise parallelen, Bioreaktorsystemen bevorzugt. Parallele Bioreaktorsysteme sind beispielsweise in der DE 10 2011 054 363.5 oder der DE 10 2011 054 365.1 beschrieben. In einem solchen Bioreaktor-System können mehrere Bioreaktoren parallel betrieben und mit hoher Genauigkeit kontrolliert werden. Dabei können auch bei kleinen Arbeitsvolumina in den einzelnen Bioreaktoren Experimente mit hohem Durchsatz durchgeführt werden, die gut reproduzierbar und skalierbar sind. Als kleine Arbeitsvolumina werden hier insbesondere Bioreaktoren mit einer Größe von bis zu 2000 ml verstanden, beispielsweise mit einem Gesamtvolumen des Reaktionsraums von etwa 350 ml bei einem Arbeitsvolumen von etwa 60 bis etwa 250 ml.

Im Anwendungsbereich der Zellkultur werden solche parallelen Bioreaktorsysteme beispielsweise für auf statistischen Planungsmethoden (Design of Experiments DoE) basierenden Versuchsreihen zur Prozessoptimierung, die Prozessentwicklung sowie die Forschung und Entwicklung eingesetzt, beispielsweise um verschiedene Zelllinien, wie Chinese Hamster Ovary (CHO)-, Hybridoma- oder NSO-Zelllinien, zu kultivieren. Unter dem Begriff "Zellkultur" wird im Rahmen des vorliegenden Textes insbesondere die Kultivierung tierischer oder pflanzlicher Zellen in einem Nährmedium außerhalb des Organismus verstanden. Die Kultivierung von (menschlichen) Stammzellen fällt ebenso unter diesen Begriff bzw. können diese Zellen ebenfalls in Bioreaktoren kultiviert werden (geschüttelt oder gerührt).

Im Anwendungsbereich der Mikrobiologie werden parallele Bioreaktorsysteme ebenfalls für auf statistischen Planungsmethoden (Design of Experiments DoE) basierenden Versuchsreihen zur Prozessoptimierung, die Prozessentwicklung sowie die Forschung und Entwicklung eingesetzt, beispielsweise um verschiedene Mikroorganismen, insbesondere Bakterien oder Pilze, z.B. Hefen, zu kultivieren.

Aufgrund von meist begrenztem Platz im Labor werden dabei geringe Platzanforderungen, insbesondere geringe Stellplatzanforderungen sowohl für Bioreaktorsysteme als auch für Bioreaktoren selbst angestrebt.

Bioreaktoren im Laboreinsatz sind oft aus Glas und/oder Metall, insbesondere rostfreiem Stahl, ausgebildet, da die Bioreaktoren zwischen verschiedenen Anwendungen sterilisiert werden müssen, was vorzugsweise durch eine Heißdampfsterilisation in einem Autoklaven erfolgt. Die Sterilisierung und Reinigung wieder verwendbarer Bioreaktoren ist aufwändig: Der Sterilisations- und Reinigungsprozess kann einer Validierung unterliegen und seine Durchführung ist für jeden einzelnen Bioreaktor genau zu dokumentieren. Rückstände in einem nicht vollständig sterilisierten Bioreaktor können die Ergebnisse eines nachfolgenden Prozesses verfälschen oder unbrauchbar machen und einen nachfolgenden Prozessablauf stören. Des Weiteren können einzelne Bestandteile bzw. Materialien der Bioreaktoren durch den Sterilisationsprozess beansprucht und z.T. beschädigt werden.

Eine Alternative zu wieder verwendbaren Bioreaktoren stellen Einweg-Bioreaktoren dar, die für die Durchführung lediglich eines biologischen bzw. biotechnologischen Prozesses verwendet und anschließend entsorgt werden. Durch die Bereitstellung eines neuen, vorzugsweise im Herstellungsprozess sterilisierten, Einweg-Bioreaktors für jeden Prozess kann die Gefahr einer (Kreuz-)Kontamination reduziert werden und gleichzeitig entfällt der Aufwand der Durchführung und Dokumentation einer einwandfreien Reinigung und Sterilisierung eines zuvor genutzten Bioreaktors. Einweg-Bioreaktoren sind oft als flexible Behälter ausgebildet, beispielsweise als Beutel oder als Behälter mit zumindest abschnittsweise flexiblen Wänden, oder als formstabile Einwegreaktoren.

Formstabile Einweg-Bioreaktoren sind oft noch relativ hochpreisig und von ihrer Konstruktion auf die pharmazeutische Prozessentwicklung und pharmazeutische Produktionsprozesse abgestimmt. Sie werden insbesondere für Zellkulturprozesse verwendet und sind demnach auch insbesondere auf solche Zellkulturprozesse konstruiert und abgestimmt. Insbesondere für Anwendungen in der Mikrobiologie gelten jedoch oft andere Anforderungen, sowohl was die am Markt erzielbaren Preise als auch geeignete Konstruktion und einsetzbare Werkstoffe angeht, um den um Größenordnungen höheren Anforderungen an prozesstechnische Parameter, wie beispielsweise Mischzeit, Energieeintrag und Gasaustausch, gerecht zu werden.

Die Erfindung beruht daher unter anderem auch auf der Erkenntnis, dass der hier beschriebene Multisensor gerade in der Verwendung mit Einweg-Bioreaktoren zur Anwendung in der Zellkultur und/oder der Mikrobiologie seine Vorteile besonders gut entfalten kann, da hier durch die Integration von mindestens drei Messanordnungen in einem Multisensor zum einen der Platzbedarf zur Messung verschiedener Parameter erheblich reduziert werden kann und gleichzeitig durch die Verwendung von elektrisch leitfähigem Kunststoff eine sehr kostengünstige Konstruktion erzielt werden kann. Durch den Einsatz eines Multisensors kann auch die Komplexität der Anschlussmaterialien reduziert werden, beispielsweise indem nur Kabel und/oder weitere Anschlussmaterialien für einen Multisensor anstatt für drei oder mehrere Einzelsensoren erforderlich ist.

Ferner können die Parameter mit dem Multisensor vorzugsweise prozessbegleitend gemessen werden, insbesondere ohne das Erfordernis einer Probennahme, bei der Fluide aus dem Reaktionsraum entnommen und außerhalb des Reaktionsraums analysiert werden müssen.

Der Multisensor kann eine Haupterstreckungsrichtung entlang einer Längsachse haben, wobei die Erstreckung des Multisensors entlang der Längsachse und/oder in Haupterstreckungsrichtung vorzugsweise um ein Vielfaches größer ist als eine Erstreckung orthogonal zur Längsachse und/oder Haupterstreckungsrichtung. Der Multisensor kann beispielsweise stabförmig und/oder zylinderförmig ausgebildet sein. Ferner kann der Multisensor orthogonal zur Längsachse und/oder Haupterstreckungsrichtung einen Querschnitt aufweisen, der kreisförmig, oval oder polygonal ausgebildet ist. Der Multisensor kann vorzugsweise ein erstes Ende und ein zweites, dem ersten gegenüberliegendes Ende aufweisen.

Die mindestens zwei Elektroden der ersten Messanordnung sind vorzugsweise an einer Oberfläche des Multisensors angeordnet und/oder derart angeordnet, dass sie beim bestimmungsgemäßen Gebrauch in einem Bioreaktor mit im Reaktionsraum des Bioreaktors befindlichen Fluiden in Kontakt kommen können.

Gemäß einer bevorzugten Ausführungsform umfasst der Multisensor eine Auswerteeinheit und/oder eine Schnittstelle zu einer, beispielsweise externen, Auswerteeinheit, wobei die Auswerteeinheit, insbesondere die Auswerteeinheit des Multisensors und/oder eine externe Auswerteeinheit, ausgebildet ist, auf Basis der Impedanzmessung Informationen über im Bioreaktor befindliche Biomasse abzuleiten, insbesondere Informationen über Zellanzahl und/oder Zellgröße und/oder Zellviabilität.

Informationen über im Bioreaktor befindliche Biomasse sind für Prozesse in der Zellkultur und/oder der Mikrobiologie besonders wichtig, weshalb die Integration einer hierfür geeigneten Impedanzmessung in den Multisensor hier besonders vorteilhaft sein kann.

Eine Impedanzmessung zwischen intrazellulären und extrazellulären Fluiden kann beispielsweise der Bestimmung der Zellgröße, insbesondere der mittleren Zellgröße, dienen. Eine Impedanzmessung zwischen Zellmembranen und Fluid kann beispielsweise der Bestimmung der Anzahl lebender Zellen und/oder Zellviabilität dienen. Eine Impedanzmessung zwischen intrazellulären und extrazellulären Fluiden und Zellmembranen mit verschiedenen Frequenzen kann beispielsweise der Bestimmung der Zellviabilität dienen. Die Impedanzmessung kommt beispielsweise auch als bioelektrische Impedanzanalyse für die Bestimmung der Körperzusammensetzung von Menschen und anderen Lebewesen zum Einsatz, beispielsweise bei Köperfettwagen.

Auch eine kapazitive Messung und/oder Füllstandsmessung und/oder Schaummessung ist für Prozesse in der Zellkultur und/oder der Mikrobiologie besonders wichtig, weshalb deren Integration in den Multisensor besonders bevorzugt sein kann. Die kapazitive Messung und/oder Füllstandsmessung und/oder Schaummessung kommt beispielsweise auch im CY8CKIT-022 CapSense® Liquid Level Sensing Shield von Cypress zum Einsatz (http://www.cypress.com/documentation/development-kitsboards/cy8ckit-022-capsense-liquid-level-sensing-shield).

Es ist ferner bevorzugt, dass eine zweite der drei Messanordnungen ausgebildet ist, eine Impedanzmessung und/oder eine kapazitive Messung und/oder eine Füllstandsmessung und/oder eine Schaummessung durchzuführen.

In einer weiteren bevorzugten Ausführungsform ist vorgesehen, dass die zweite Messanordnung mindestens zwei Elektroden aufweist, die einen elektrisch leitfähigen Kunststoff umfassen oder daraus bestehen.

Die mindestens zwei Elektroden der ersten und/oder die mindestens zwei Elektroden der zweiten Messanordnung können beispielsweise unterhalb der Oberfläche des Multisensors angeordnet und/oder derart angeordnet sein, dass sie beim bestimmungsgemäßen Gebrauch in einem Bioreaktoren mit im Reaktionsraum des Bioreaktors befindlichen Fluiden nicht unmittelbar in Kontakt kommen.

Eine bevorzugte Weiterbildung zeichnet sich dadurch aus, dass eine dritte der drei Messanordnungen ausgebildet ist, eine Temperaturmessung durchzuführen. Ferner vorzugsweise weist die dritte Messanordnung mindestens ein Messelement für die Temperaturmessung auf. Das Messelement für die Temperaturmessung kann beispielsweise als Widerstandsthermometer ausgebildet sein.

Die erste und/oder die zweite und/oder die dritte Messanordnung können vorzugsweise zwei oder mehrere Elektroden aufweisen, welche ganz oder teilweise aus elektrisch leitfähigem Kunststoff bestehen oder elektrisch leitfähigen Kunststoff aufweisen. Ein leitfähiger Kunststoff kann beispielsweise ein Kunststoff mit einem leitfähigen Additiv oder ein intrinsisch leitfähiger Kunststoff sein. Als leitfähige Kunststoffe können insbesondere Polymere wie Polypropylen mit einem leitfähigen Additiv (z.B. Leitruß, Kohlefasern, Karbonnanotubes, Metallpulver oder -fasern, niedrig schmelzende Legierungen) oder intrinsisch leitfähige Polymere (z.B. Polyanilin, Polythiophen oder Polypyrrol) eingesetzt werden.

In einer bevorzugten Ausführungsform ist vorgesehen, dass die erste und/oder die zweite und/oder die dritte Messanordnung eine, zwei oder mehrere Isolationsabschnitte aufweist, welche ganz oder teilweise aus nicht elektrisch leitfähigem und/oder isolierendem Kunststoff bestehen oder nicht elektrisch leitfähigen und/oder isolierenden Kunststoff aufweisen. Die Isolationsabschnitte können zwischen und/oder neben Elektroden angeordnet sein. Die Isolationsabschnitte können auch auf und/oder unter Elektroden angeordnet sein, beispielsweise um einen direkten Kontakt der Elektroden mit den Multisensor umgebenden Fluiden zu verhindern und eine schützende Außenoberfläche zu bilden.

Als nicht elektrisch leitfähiger und/oder isolierender Kunststoff kann insbesondere Polyolefin eingesetzt werden, z.B. Polypropylen, Polyethylen oder ein Blend aus beiden.

Bevorzugt enthalten die Elektroden und die Isolationsabschnitte den gleichen Kunststoff, wodurch eine stoffschlüssige Verbindung gefördert wird. Besonders bevorzugt werden ein elektrisch leitfähiges Polypropylen für die Elektrode und ein nicht elektrisch leitfähiges und/oder isolierendes Polypropylen für den Isolierabschnitt eingesetzt.

Vorzugsweise werden Materialien und/oder eine Materialkombination gewählt, die den Anforderungen der United States Pharmacopeia (USP) Klasse VI entspricht bzw. entsprechen. Insbesondere ist es bevorzugt, dass der elektrisch leitfähige Kunststoff und/oder der nicht elektrisch leitfähige und/oder isolierende Kunststoff den Anforderungen der United States Pharmacopeia (USP) Klasse VI entspricht.

Es ist ferner bevorzugt, dass die erste und/oder die zweite und/oder die dritte Messanordnung ganz oder teilweise durch Urformen hergestellt ist. In einer bevorzugten Ausführungsform ist vorgesehen, dass eine, zwei oder mehrere Elektroden der ersten und/oder der zweiten und/oder der dritten Messanordnung durch Urformen hergestellt sind. Es ist ferner bevorzugt, dass ein, zwei oder mehrere Isolierabschnitte der ersten und/oder der zweiten und/oder der dritten Messanordnung durch Urformen hergestellt sind. Vorzugsweise kann auch vorgesehen sein, dass eine, zwei oder mehrere Elektroden der ersten und/oder der zweiten und/oder der dritten Messanordnung und ein, zwei oder mehrere Isolierabschnitte der ersten und/oder der zweiten Messanordnung durch Urformen hergestellt sind.

Es ist insbesondere bevorzugt, dass die erste und/oder die zweite und/oder die dritte Messanordnung ganz oder teilweise durch Spritzgießen hergestellt ist und/oder die erste und/oder die zweite und/oder die dritte Messanordnung ganz oder teilweise durch Mehrkomponenten-Spritzgießen hergestellt ist. In einer bevorzugten Ausführungsform ist vorgesehen, dass eine, zwei oder mehrere Elektroden der ersten und/oder der zweiten und/oder der dritten Messanordnung durch Spritzgießen hergestellt sind. Es ist ferner bevorzugt, dass ein, zwei oder mehrere Isolierabschnitte der ersten und/oder der zweiten und/oder der dritten Messanordnung durch Spritzgießen hergestellt sind. Vorzugsweise kann auch vorgesehen sein, dass eine, zwei oder mehrere Elektroden der ersten und/oder der zweiten und/oder der dritten Messanordnung und ein, zwei oder mehrere Isolierabschnitte der ersten und/oder der zweiten Messanordnung durch Mehrkomponenten-Spritzgießen hergestellt sind.

Es ist insbesondere bevorzugt, dass die erste und/oder die zweite und/oder die dritte Messanordnung ganz oder teilweise durch additives Fertigen hergestellt ist. In einer bevorzugten Ausführungsform ist vorgesehen, dass eine, zwei oder mehrere Elektroden der ersten und/oder der zweiten und/oder der dritten Messanordnung durch additives Fertigen hergestellt sind. Es ist ferner bevorzugt, dass ein, zwei oder mehrere Isolierabschnitte der ersten und/oder der zweiten und/oder der dritten Messanordnung durch additives Fertigen hergestellt sind. Vorzugsweise kann auch vorgesehen sein, dass eine, zwei oder mehrere Elektroden der ersten und/oder der zweiten und/oder der dritten Messanordnung und ein, zwei oder mehrere Isolierabschnitte der ersten und/oder der zweiten Messanordnung durch additives Fertigen hergestellt sind.

Unter Urformen werden hier insbesondere Fertigungsverfahren verstanden, bei denen aus einem formlosen Stoff ein fester Körper hergestellt wird, der eine geometrisch definierte Form hat.

Beispiele für Urformverfahren sind Spritzgießen und/oder Mehrkomponenten-Spritzgießen. Spritzgießen und/oder Mehrkomponenten-Spritzgießen kann z.B. auch Umspritzen und/oder Sequenzumspritzen und/oder Insert-Spritzgießen und/oder Outsert-Spritzgießen und/oder Hybrid-Spritzgießen umfassen,

Unter Mehrkomponenten-Spritzgießen wird hier insbesondere auch Zweikomponenten-Spritzgießen verstanden. Mehrkomponentenspritzguss beschreibt insbesondere das Herstellen von Spritzgussteilen aus zwei oder mehreren verschiedenen Kunststoffen oder Materialien und kann im Verbundspritzgießen und/oder im Montagespritzgießen und/oder im Sandwich-Spritzgießen eingesetzt werden. In einem Mehrkomponenten-Spritzgießverfahren kann lediglich Spritzgießwerkzeug oder zwei oder mehrere Spritzgießwerkzeuge zum Einsatz kommen.

Ein Mehrkomponenten-Spritzgußverfahren kann auf verschiedene Weise ausgeführt werden. Beim Kernrückzug-Verfahren wird nach dem Spritzen und Erstarren der ersten Kunststoffkomponente durch Zurückziehen eines oder mehrerer Elemente eines Formnestes ein weiterer Hohlraum freigegeben, in den die zweite Kunststoffkomponente eingespritzt wird, wobei die Form aber geschlossen bleibt. Beim Umsetz-Verfahren wird nach dem Spritzen und Erstarren der ersten Kunststoffkomponente diese bei geöffnetem Werkzeug in eine neue Kavität eingesetzt, die entsprechende Aussparungen für die zweite Kunststoffkomponente aufweist. Dies kann mit einem Handlingsystem geschehen, wobei die zweite Kavität im gleichen Werkzeug oder sogar in einer getrennten Form und auf einer zweiten Maschine liegen kann. Eine Zwischenlösung ist das Umsetzen in Indexplatten-, Drehteller- oder Etagenwende-Werkzeugen, bei denen der Vorspritzling entweder auf dem Kern oder in dem Formnest der einen Formhälfte verbleibt und bei geöffnetem Werkzeug durch Drehung eines Werkzeugbereichs in eine zweite Kavität umgesetzt wird. Diese Lösung bedingt den Zusammenhalt des Vorspritzlings, der auf der Nadel verbleibt.

Ein weiteres Beispiel für ein Urformverfahren ist das additive Fertigen, das auch als generatives Fertigen oder 3D-Druck bezeichnet wird. Ein Beispiel für das additive Fertigen unter Einsatz von Kunststoffen ist das FFF (Fused Filament Fabrication) - Verfahren oder auch FDM (Fused Deposition Modeling) -Verfahren.

Der Multisensor kann auch eine oder mehrere weitere Messanordnungen umfassen. Diese weiteren eine oder mehrere weitere Messanordnungen können ganz oder teilweise so wie die erste und/oder die zweite Messanordnung und/oder die dritte Messanordnung oder ganz oder teilweise anders ausgebildet sein.

Die Herstellung im Spritzgießverfahren und/oder im Mehrkomponenten-Spritzgießverfahren hat neben dem Vorteil einer kostengünstigen Herstellung auch den Vorteil, dass die Elemente einstückig und/oder integral gefertigt werden können, so dass Fügestellen und/oder Zwischenräume und/oder Anschlussstellen vermieden oder reduziert werden können. Auf diese Weise kann beispielsweise auch der Einsatz von Klebstoff reduziert oder vermieden werden, wodurch auch die Gefahr der Kontamination des Reaktionsraums eines Bioreaktors weiter verringert werden kann.

Der Multisensor ist vorzugsweise als Einweg-Multisensor ausgebildet. Ein Einweg-Multisensor zeichnet sich insbesondere dadurch aus, dass er für einen einmaligen Gebrauch vorgesehen ist. Hierfür kann der Multisensor ausgebildet sein, dass er nach dem einmaligen Gebrauch für einen weiteren Gebrauch nicht mehr geeignet ist. Dies kann beispielsweise so realisiert sein, dass der Multisensor ganz oder teilweise aus Materialien ausgebildet ist, die einen für eine Wiederverwendung erforderlichen Sterilisierungsprozess nicht unbeschadet überstehen, da beispielsweise die bei der Stabilisierung auftretenden Temperaturen die Materialien ganz oder teilweise zerstören oder verformen. Der Multisensor kann beispielsweise auch Warnhinweise und/oder Verwendungsangaben enthalten, welche eine mehrfache Verwendung ausschließen. Auch kann der mechanische und/oder elektrische Anschluss derart ausgeführt sein, dass er nur eine einmalige Verwendung ermöglicht.

Eine weitere Ausprägung kann darin bestehen, dass die (im Betriebszustand) im Bioreaktor befindlichen Sensorelemente als Einwegeinheiten (Elektronik Umhüllende), die Messelektronik aber als Mehrwegeinheit ausgeführt ist. Vorzugsweise wird die Messelektronik während dem Versuch in die umhüllende Sensoreinheit eingeführt, arretiert und nach dem Versuch in ein neues Einweggefäß mit einer weiteren umhüllenden Sensoreinheit transferiert oder gelagert.

Der Multisensor kann als separates Element ausgebildet sein, das beispielsweise in ein Gefäß und/oder einen Bioreaktor eingebracht und/oder daran angeschlossen werden kann und vorzugsweise wieder daraus entnommen und/oder davon entfernt werden kann.

Der Multisensor kann einstückig ausgebildet sein oder zwei oder mehrere Module umfassen, die miteinander lösbar oder nicht lösbar verbunden sind. Eine einstückige Ausbildung des Multisensors kann vorzugsweise durch Urformen erhalten werden.

Der Multisensor kann auch zwei oder mehrere Module umfassen, wobei ein Module beispielsweise eine Messanordnung umfassen kann. Ein Modul kann auch zwei oder mehr Messanordnungen umfassen. Ein Modul kann auch einen, zwei oder mehrere Teile einer Messanordnung umfassen. Zwei oder mehrere Module können mit einander verbunden sein, beispielsweise über eine Steckverbindung. Die Verbindung kann lösbar oder nicht lösbar, insbesondere nicht zerstörungsfrei lösbar, ausgebildet sein. Die Verbindungen unterschiedlicher Module zueinander können verschieden ausgebildet sein. Die verschiedenen Module können jeweils durch Urformen erhalten werden.

Die zwei oder mehreren Module können auch als ein Basismodul und ein oder mehrere Erweiterungsmodule ausgebildet sein. Vorzugsweise umfasst das Basismodul einen (weiter unten noch näher beschriebenen) Anschlusskopf und/oder eine Auswerteeinheit und/oder eine Schnittstelle zu einer Auswerteeinheit und/oder eine oder mehrere weitere Elemente des Multisensors. Ein Erweiterungsmodul umfasst vorzugsweise eine, zwei oder mehrere Messanordnungen oder Teile davon.

Ein modularer Aufbau des Multisensors hat unter anderem den Vorteil, dass mit geringem Aufwand verschiedene Multisensoren beispielsweise mit unterschiedlichen Messanordnungen hergestellt werden können und damit flexibel auf Kundenanforderungen eingegangen werden kann.

Eine bevorzugte Weiterbildung zeichnet sich dadurch aus, dass der Multisensor einen Anschlusskopf umfasst, der an einer Anschlusseinrichtung des Bioreaktors befestigbar ist. Der Anschlusskopf kann beispielsweise ein Gewinde, insbesondere ein Innengewinde und/oder ein Außengewinde, aufweisen, um mit einem korrespondierenden Gewinde der Anschlusseinrichtung des Bioreaktors zusammenzuwirken. Der Anschlusskopf ist vorzugsweise an einem ersten Ende des Multisensors angeordnet. Der Anschlusskopf kann ferner eine Schnittstelle aufweisen, insbesondere eine Schnittstelle zu einer Auswerteeinheit, insbesondere einer externen Auswerteeinheit. Die Schnittstelle kann vorzugsweise für eine elektrische und/oder Kommunikationsverbindung ausgebildet sein.

Vorzugsweise umfasst der Multisensor eine oder mehrere weitere Messanordnungen, insbesondere zur Messung weiterer Parameter, wie beispielsweise pH-Wert und/oder Gelöstsauerstoff und/oder Kohlendioxidgehalt und/oder Edukt-/Produkt oder Metabolitkonzentrationen wie z.B.: Glucose, Glutamat, Glutamin, Ammonium, etc.. Eine oder mehrere weitere Messanordnungen können vorzugsweise an einem zweiten Ende des Multisensors angeordnet sein. Die eine oder mehreren weiteren Messanordnungen können vorzugsweise eine oder mehrere Elektroden aufweisen und/oder elektrisch leitfähigen Kunststoff und/oder nicht elektrisch leitfähigen und/oder isolierenden Kunststoff umfassen oder aus einem oder mehreren solcher Materialien bestehen. Die Integration von mehr als drei Messanordnungen in einem Multisensor hat den Vorteil, dass weiterer Platzbedarf eingespart werden kann. Ferner können bei einer kostengünstigen Konstruktion des Multisensors Kosten für die Bereitstellung weiterer Einzelsensoren entfallen.

Weitere vorteilhafte Ausführungsvarianten der erfindungsgemäßen Vorrichtung ergeben sich durch Kombination der hier erörterten bevorzugten Merkmale.

Gemäß einem weiteren Aspekt der Erfindung wird die eingangs genannte Aufgabe gelöst durch einen Bioreaktor zur Anwendung in der Zellkultur und/oder der Mikrobiologie umfassend einen zuvor beschriebenen Multisensor.

Der Bioreaktor ist vorzugsweise als Einweg-Bioreaktor ausgebildet. Ein Einweg-Bioreaktor zeichnet sich insbesondere dadurch aus, dass er für einen einmaligen Gebrauch vorgesehen ist. Hierfür kann der Bioreaktor ausgebildet sein, dass er nach dem einmaligen Gebrauch für einen weiteren Gebrauch nicht mehr geeignet ist. Dies kann beispielsweise so realisiert sein, dass der Bioreaktor ganz oder teilweise aus Materialien ausgebildet ist, die einen für eine Wiederverwendung erforderlichen Sterilisierungsprozess nicht unbeschadet überstehen, da beispielsweise die bei der Stabilisierung auftretenden Temperaturen die Materialien ganz oder teilweise zerstören oder verformen. Der Bioreaktor kann beispielsweise auch Warnhinweise und/oder Verwendungsangaben enthalten, welche eine mehrfache Verwendung ausschließen.

Gemäß einem weiteren Aspekt der Erfindung wird die eingangs genannte Aufgabe gelöst durch ein Verfahren zur Herstellung eines zuvor beschriebenen Multisensors, das Verfahren umfassend Integrieren von mindestens drei Messanordnungen in einen Multisensor, wobei eine erste der drei Messanordnungen ausgebildet ist, eine Impedanzmessung und/oder eine kapazitive Messung durchzuführen, und wobei die erste Messanordnung mindestens zwei Elektroden aufweist, die einen elektrisch leitfähigen Kunststoff umfassen oder daraus bestehen.

Das Verfahren zur Herstellung eines zuvor beschriebenen Multisensors umfasst ferner vorzugsweise
- Urformen der ersten und/oder der zweiten und/oder der dritten Messanordnung ganz oder teilweise, und/oder
- Urformen von einer, zwei oder mehreren Elektroden der ersten und/oder der zweiten und/oder der dritten Messanordnung ganz oder teilweise aus elektrisch leitfähigem Kunststoff, insbesondere elektrisch leitfähigem Polypropylen, und/oder
- Urformen von einem, zwei oder mehreren Isolierabschnitten der ersten und/oder der zweiten und/oder der dritten Messanordnung ganz oder teilweise ganz oder teilweise aus nicht elektrisch leitfähigem und/oder isolierendem Kunststoff, und/oder
- Urformen von einer, zwei oder mehreren Elektroden der ersten und/oder der zweiten und/oder der dritten Messanordnung und von einem, zwei oder mehreren Isolierabschnitten der ersten Messanordnung,

Das Verfahren zur Herstellung eines zuvor beschriebenen Multisensors umfasst insbesondere
- Spritzgießen der ersten und/oder der zweiten und/oder der dritten Messanordnung ganz oder teilweise und/oder Mehrkomponenten-Spritzgießen der ersten und/oder der zweiten und/oder der dritten Messanordnung ganz oder teilweise, und/oder
- Spritzgießen von einer, zwei oder mehreren Elektroden der ersten und/oder der zweiten und/oder der dritten Messanordnung ganz oder teilweise aus elektrisch leitfähigem Kunststoff, insbesondere elektrisch leitfähigem Polypropylen, und/oder
- Spritzgießen von einem, zwei oder mehreren Isolierabschnitten der ersten und/oder der zweiten und/oder der dritten Messanordnung ganz oder teilweise ganz oder teilweise aus nicht elektrisch leitfähigem und/oder isolierendem Kunststoff, und/oder
- Mehrkomponenten-Spritzgießen von einer, zwei oder mehreren Elektroden der ersten und/oder der zweiten und/oder der dritten Messanordnung und von einem, zwei oder mehreren Isolierabschnitten der ersten Messanordnung,
- Additives Fertigen der ersten und/oder der zweiten und/oder der dritten Messanordnung ganz oder teilweise, und/oder
- Additives Fertigen von einer, zwei oder mehreren Elektroden der ersten und/oder der zweiten und/oder der dritten Messanordnung ganz oder teilweise aus elektrisch leitfähigem Kunststoff, insbesondere elektrisch leitfähigem Polypropylen, und/oder
- Additives Fertigen von einem, zwei oder mehreren Isolierabschnitten der ersten und/oder der zweiten und/oder der dritten Messanordnung ganz oder teilweise ganz oder teilweise aus nicht elektrisch leitfähigem und/oder isolierendem Kunststoff, und/oder
- Additives Fertigen von einer, zwei oder mehreren Elektroden der ersten und/oder der zweiten und/oder der dritten Messanordnung und von einem, zwei oder mehreren Isolierabschnitten der ersten Messanordnung.

Gemäß einem weiteren Aspekt der Erfindung wird die eingangs genannte Aufgabe gelöst durch die Verwendung eines zuvor beschriebenen Multisensors zur Messung von mindestens drei Parametern in einem Bioreaktor zur Anwendung in der Zellkultur und/oder der Mikrobiologie.

Gemäß einem weiteren Aspekt der Erfindung wird die eingangs genannte Aufgabe gelöst durch ein Verfahren zur Messung von mindestens drei Parametern in einem Bioreaktor zur Anwendung in der Zellkultur und/oder der Mikrobiologie, das Verfahren umfassend,
- Bereitstellen eines zuvor beschriebenen Multisensors,
- Durchführen einer Impedanzmessung und/oder einer kapazitiven Messung mit einer ersten der drei Messanordnungen,
- Durchführen von zwei weiteren Messungen mit der zweiten und dritten der drei Messanordnungen.

Zu den Vorteilen, Ausführungsvarianten und Ausführungsdetails der weiteren Aspekte der Erfindung und ihrer Fortbildungen wird auf die vorangegangene Beschreibung zu den entsprechenden Merkmalen des Multisensors verwiesen.

Die hier beschriebenen Verfahren zur Herstellung eines zuvor beschriebenen Multisensors und zur Messung von mindestens drei Parametern in einem Bioreaktor zur Anwendung in der Zellkultur und/oder der Mikrobiologie sowie ihre jeweiligen Fortbildungen weisen insbesondere Merkmale bzw. Verfahrensschritte auf, die sie dafür geeignet machen, für einen und/oder mit einem hier beschriebenen Multisensor und seinen Fortbildungen verwendet zu werden.

Bevorzugte Ausführungsformen der Erfindung werden beispielhaft anhand der beiliegenden Figuren beschrieben. Es zeigen:
- Figur 1:: eine dreidimensionale Darstellung eines Multisensors;
- Figur 2:: eine dreidimensionale Darstellung eines Teils eines Multisensors mit einer ersten Messanordnung;
- Figur 3:: eine dreidimensionale Darstellung eines Teils eines Multisensors mit einem Abschnitt einer zweiten Messanordnung; und
- Figur 4:: eine dreidimensionale Darstellung eines Einweg-Bioreaktors mit einem Multisensor.

Ähnliche oder im Wesentlichen funktionsgleiche Elemente werden in den Figuren mit den gleichen Bezugszeichen bezeichnet.

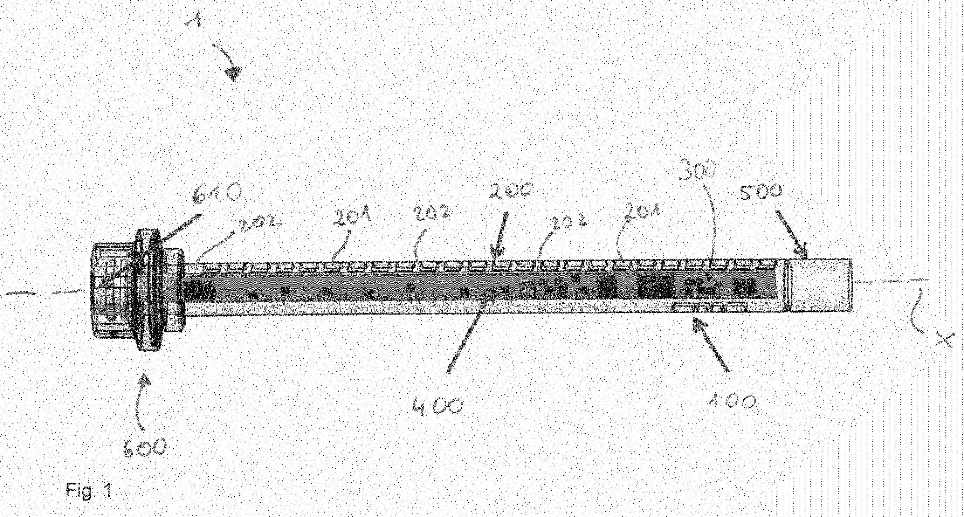

Fig. 1 zeigt eine dreidimensionale Darstellung einer Ausprägung eines Multisensors 1. Der hier gezeigte Multisensor 1 weist eine Haupterstreckungsrichtung entlang der Längsachse X auf, wobei die Erstreckung des Multisensors 1 entlang der Längsachse X um ein Vielfaches größer ist als eine Erstreckung orthogonal zur Längsachse. Der Multisensor 1 ist im hier gezeigten Beispiel stabförmig und ausgebildet und hat im Wesentlichen die Form eines Zylinders. Der Querschnitt des Multisensors orthogonal zur Längsachse und Haupterstreckungsrichtung weist einen kreisförmigen Querschnitt auf.

An einem ersten Ende des Multisensors 1 ist ein Anschlusskopf 600 angeordnet mit einer Schnittstelle 610, die vorzugsweise für elektrische und/oder Kommunikationsverbindungen geeignet ist.

Der Multisensor 1 weist eine erste Messanordnung 100 auf, die für eine Impedanzmessung ausgebildet ist. Ferner weist der Multisensor 1 eine zweite Messanordnung 200 auf, die ausgebildet ist, eine kapazitive Messung und/oder eine Füllstandsmessung und/oder eine Schaummessung durchzuführen. Der Multisensor 1 weist ferner eine dritte Messanordnung 300 auf, die ausgebildet ist, eine Temperaturmessung durchzuführen.

Der Multisensor 1 kann darüber hinaus auch weitere Messanordnungen aufweisen zur Messung weiterer Parameter, wie beispielsweise pH-Wert und/oder Gelöstsauerstoff und/oder Kohlendioxidgehalt und/oder Edukt-/Produkt oder Metabolitkonzentrationen wie z.B.: Glucose, Glutamat, Glutamin, Ammonium, etc., die beispielsweise an einem zweiten, dem ersten gegenüberliegenden Ende 500 des Multisensors 1 angeordnet sein können.

Die dritte Messanordnung 300 ist an einem Bauelement 400 mit einer integrierten Elektronik mit einem Mikrocontroller und einem analogen Frontend angeordnet. Die integrierte Elektronik des Bauelements 400 kann als Auswerteeinheit dienen, gegebenenfalls auch zusammen mit einer über die im Anschlusskopf 600 angeordnete Schnittstelle verbundenen externen Auswerteeinheit.

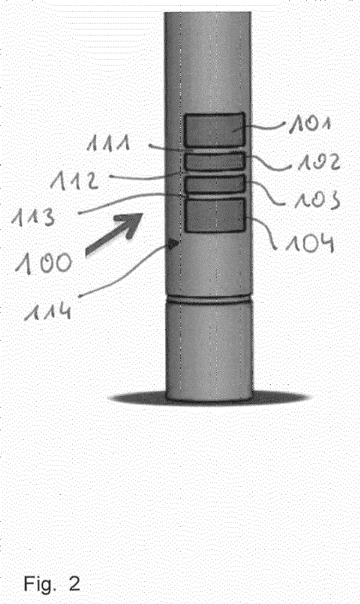

In Figur 2 ist ein Teil einer möglichen Ausprägung eines Multisensors mit einer ersten Messanordnung 100 vergrößert dargestellt. Die erste Messanordnung 100 umfasst vorzugsweise vier Elektroden 101, 102, 103, 104, die jeweils aus elektrisch leitfähigem Kunststoff ausgebildet sind oder elektrisch leitfähigen Kunststoff umfassen. Getrennt und/oder umgeben sind die Elektroden 101, 102, 103, 104 von Isolationsabschnitten 111, 112, 113, 114, welche aus nicht elektrisch leitfähigem und/oder isolierendem Kunststoff bestehen oder einen solchen aufweisen. Die erste Messanordnung 100 ist ausgebildet, eine Impedanzmessung durchzuführen. Die Elektroden 101, 102, 103, 104 sind in Haupterstreckungsrichtung des Multisensors 1 äquidistant voneinander beabstandet. Die Elektroden 101 und 104 weisen in Haupterstreckungsrichtung des Multisensors 1 eine größere Erstreckung auf als die beiden Elektroden 102 und 103. Die Elektroden 101, 102, 103, 104 sind an einer Oberfläche des Multisensors 1 angeordnet und derart angeordnet, dass sie beim bestimmungsgemäßen Gebrauch in einem Bioreaktor mit im Reaktionsraum des Bioreaktors befindlichen Fluiden in Kontakt kommen.

Vorzugsweise ist eine Auswerteeinheit des Multisensors 1 vorgesehen und/oder eine Schnittstelle 600 des Multisensors 1 zu einer Auswerteeinheit, wobei diese Auswerteeinheit ausgebildet ist, auf Basis der Impedanzmessung Informationen über im Bioreaktor befindliche Biomasse abzuleiten, insbesondere Informationen über Zellanzahl und/oder Zellgröße und/oder Zellviabilität.

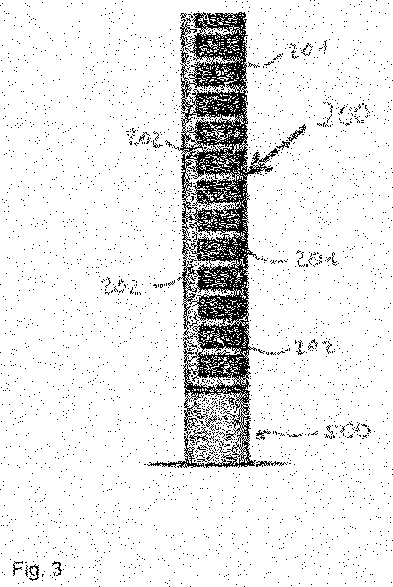

In Figur 3 ist ein Teil eines Multisensors mit einem Abschnitt einer zweiten Messanordnung 200 vergrößert dargestellt. Die zweite Messanordnung 200 ist ausgebildet, eine kapazitive Messung und/oder eine Füllstandsmessung und/oder eine Schaummessung durchzuführen. Ferner weist die zweite Messanordnung 200 mehrere, im hier dargestellten Beispiel in Haupterstreckungsrichtung des Multisensors 1 äquidistant beabstandete, Elektroden 201 auf, die einen elektrisch leitfähigen Kunststoff umfassen oder daraus bestehen. Auch diese Elektroden 201 sind durch Isolationsabschnitte 202 getrennt und/oder von Isolationsabschnitten 202 umgeben, wobei die Isolationsabschnitte 202 aus nicht elektrisch leitfähigem und/oder isolierendem Kunststoff bestehen oder einen solchen aufweisen. Über die Anordnung, insbesondere die Beabstandung entlang der Längsachse X, der Elektroden der zweiten Messanordnung 200 kann die Auflösung der Füllstandsmessung und/oder Schaummessung beeinflusst werden. Die Elektroden 201 sind unterhalb der Oberfläche des Multisensors angeordnet und derart angeordnet, dass sie beim bestimmungsgemäßen Gebrauch in einem Bioreaktoren mit im Reaktionsraum des Bioreaktors befindlichen Fluiden nicht unmittelbar in Kontakt kommen. Hierzu sind vorzugsweise Isolationsabschnitte auch auf den Elektroden 201 ausgebildet, um einen direkten Kontakt der Elektroden 201 mit den Multisensor 1 umgebenden Fluiden zu verhindern und eine schützende Außenoberfläche zu bilden.

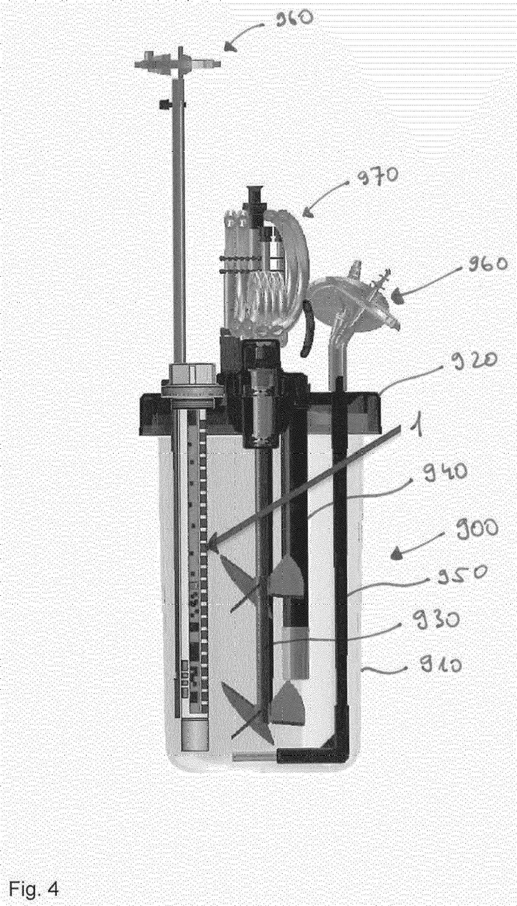

In Figur 4 ist der Multisensor 1 in einem Einweg-Bioreaktor 900 angeordnet zu sehen. Der Einweg-Bioreaktor 900 weist eine Kopfplatte 920, einen formstabilen Behälter 910 und ein Rührwerk 930 auf. Die Kopfplatte 920 und der Behälter 910 schließen einen Reaktionsraum ein. Die Kopfplatte 920 hat eine dem Reaktionsraum zugewandte Innenseite, an der mehrere Tauchrohre 940, 950 angeordnet sind, die in den Reaktionsraum ragen. Auf einer dem Reaktionsraum abgewandten Außenseite der Kopfplatte 920 sind mehrere Anschlüsse angeordnet, an denen Schläuche und Anschlussmaterialien 970 und Sterilfilter 960 angeordnet sind.

Der Multisensor 1 ist im Einbauzustand im Einweg-Bioreaktor 900 im wesentlichen vertikal ausgerichtet angeordnet, sodass der Anschlusskopf 600 des Multisensors 1 an der Kopfplatte 920 des Einweg-Bioreaktors 900 angeordnet ist und der Multisensor 1 von dort aus entlang seiner Haupterstreckungsrichtung in den Reaktionsraum des Einweg-Bioreaktors 900 ragt.

Das Rührwerk 930 weist eine Rührwelle 310 mit einer Rotationsachse und zwei Rührelementen auf, die hier mit um 45° geneigten Flügeln ausgebildet sind, beispielsweise als Pitch Blade Impeller. Alternativ kann beispielsweise auch mindestens ein Rushton-Impeller als Rührelement eingesetzt werden. Die Rührelemente sind drehsteif an der Rührwelle befestigt, sodass bei einer Rotation der Rührwelle auf die Rührelemente mitrotieren.

Die Kopfplatte 920 und der Behälter 910 können beispielsweise aus Polyamid ausgebildet sein oder Polyamid umfassen und mittels Ultraschallschweißen miteinander unlösbar verbunden sein. Das Rührwerk 930, insbesondere die Rührwelle und/oder die Rührelemente, können beispielsweise aus Polystyrol ausgebildet sein oder Polystyrol umfassen.

Mit dem Einweg-Bioreaktor 900 verwendete Schläuche und Anschlussmaterialien 970, die mit Reaktionsmedien in Berührung kommen können, sind vorzugsweise aus Materialien ausgebildet, die nach der United States Pharmacopeia (USP) Klasse VI zertifiziert sind, wie beispielsweise Polystyrol, Polycarbonat, Polyamid oder Silikon. Die einzusetzenden Schläuche sind vorzugsweise flexible Schläuche aus thermoplastischen Elastomeren.

Durch den Einsatz eines Multisensors 1 im Einweg-Bioreaktor 900 kann ein Anschluss an der Kopfplatte 920 für die Messung von drei (oder mehr) Parametern genutzt werden. Wie in Figur 4 beispielhaft zu erkennen ist, ist der Raum auf der Kopfplatte begrenzt, wobei gleichzeitig eine Vielzahl von Elementen anzuschließen sind. Die Integration von drei Sensoren in einen Multisensor ist somit besonders vorteilhaft, insbesondere, wenn die erste Messanordnung für eine Impedanzmessung und/oder eine kapazitive Messung geeignet ist.

Durch den Einsatz von elektrisch leitfähigem Kunststoff bei den Elektroden kann zudem eine kostengünstige Konstruktion für den Multisensor erzielt werden, was insbesondere auch dessen Ausbildung als Einweg-Multisensor ermöglicht. Auf diese Weise können weitere Anwendungsbereiche erschlossen werden.

## Patentansprüche

1. Multisensor für einen Bioreaktor zur Anwendung in der Zellkultur und/oder der Mikrobiologie, der Multisensor umfassend
- mindestens drei Messanordnungen, die ausgebildet sind, mindestens drei Parameter zu messen,
- wobei eine erste der drei Messanordnungen ausgebildet ist, eine Impedanzmessung und/oder eine kapazitive Messung durchzuführen, und
- wobei die erste Messanordnung mindestens zwei Elektroden aufweist, die einen elektrisch leitfähigen Kunststoff umfassen oder daraus bestehen.

2. Multisensor nach mindestens einem der vorhergehenden Ansprüche, umfassend eine Auswerteeinheit und/oder eine Schnittstelle zu einer Auswerteeinheit, wobei die Auswerteeinheit ausgebildet ist, auf Basis einer Impedanzmessung Informationen über im Bioreaktor befindliche Biomasse abzuleiten, insbesondere Informationen über Zellanzahl und/oder Zellgröße und/oder Zellviabilität.

3. Multisensor nach mindestens einem der vorhergehenden Ansprüche, **dadurch gekennzeichnet, dass** eine zweite der drei Messanordnungen ausgebildet ist, eine Impedanzmessung und/oder eine kapazitive Messung und/oder eine Füllstandsmessung und/oder eine Schaummessung durchzuführen.

4. Multisensor nach mindestens einem der vorhergehenden Ansprüche, **dadurch gekennzeichnet, dass** die zweite Messanordnung mindestens zwei Elektroden aufweist, die einen elektrisch leitfähigen Kunststoff umfassen oder daraus bestehen.

5. Multisensor nach mindestens einem der vorhergehenden Ansprüche, **dadurch gekennzeichnet, dass** eine dritte der drei Messanordnungen ausgebildet ist, eine Temperaturmessung durchzuführen.

6. Multisensor nach mindestens einem der vorhergehenden Ansprüche, **dadurch gekennzeichnet, dass** die erste und/oder die zweite und/oder die dritte Messanordnung eine, zwei oder mehrere Isolationsabschnitte aufweist, welche ganz oder teilweise aus nicht elektrisch leitfähigem und/oder isolierendem Kunststoff bestehen oder nicht elektrisch leitfähigen und/oder isolierenden Kunststoff aufweisen.

7. Multisensor nach mindestens einem der vorhergehenden Ansprüche, **dadurch gekennzeichnet, dass**
- die erste und/oder die zweite und/oder die dritte Messanordnung ganz oder teilweise durch Urformen hergestellt ist, und/oder
- eine, zwei oder mehrere Elektroden der ersten und/oder der zweiten und/oder der dritten Messanordnung durch Urformen hergestellt sind, und/oder
- ein, zwei oder mehrere Isolierabschnitte der ersten und/oder der zweiten und/oder der dritten Messanordnung durch Urformen hergestellt sind, und/oder
- eine, zwei oder mehrere Elektroden der ersten und/oder der zweiten und/oder der dritten Messanordnung und ein, zwei oder mehrere Isolierabschnitte der ersten und/oder der zweiten Messanordnung durch Urformen hergestellt sind.

8. Multisensor nach mindestens einem der vorhergehenden Ansprüche, **dadurch gekennzeichnet, dass** der Multisensor als Einweg-Multisensor ausgebildet ist.

9. Multisensor nach mindestens einem der vorhergehenden Ansprüche, **dadurch gekennzeichnet, dass** der Multisensor einstückig ausgebildet ist oder zwei oder mehrere Module umfasst, die miteinander lösbar oder nicht lösbar verbunden sind.

10. Multisensor nach mindestens einem der vorhergehenden Ansprüche, **dadurch gekennzeichnet, dass** in einem Bioreaktor befindliche Sensorelemente als Einwegeinheiten ausgebildet sind und eine Messelektronik als Mehrwegeinheit ausgebildet ist.

11. Multisensor nach mindestens einem der vorhergehenden Ansprüche, umfassend einen Anschlusskopf, der an einer Anschlusseinrichtung des Bioreaktors befestigbar ist.

12. Multisensor nach mindestens einem der vorhergehenden Ansprüche, umfassend eine oder mehrere weitere Messanordnungen, insbesondere zur Messung weiterer Parameter, wie beispielsweise pH-Wert und/oder Gelöstsauerstoff und/oder Kohlendioxidgehalt und/oder Edukt-/Produkt oder Metabolitkonzentrationen wie z.B.: Glucose, Glutamat, Glutamin, Ammonium, etc..

13. Bioreaktor zur Anwendung in der Zellkultur und/oder der Mikrobiologie umfassend einen Multisensor nach mindestens einem der vorhergehenden Ansprüche.

14. Bioreaktor nach mindestens einem der vorhergehenden Ansprüche, wobei der Bioreaktor als Einweg-Bioreaktor ausgebildet ist

15. Verfahren zur Herstellung eines Multisensors nach mindestens einem der vorhergehenden Ansprüche, das Verfahren umfassend
- Integrieren von mindestens drei Messanordnungen in einen Multisensor, wobei eine erste der drei Messanordnungen ausgebildet ist, eine Impedanzmessung und/oder eine kapazitive Messung durchzuführen, und wobei die erste Messanordnung mindestens zwei Elektroden aufweist, die einen elektrisch leitfähigen Kunststoff umfassen oder daraus bestehen.

16. Verfahren nach mindestens einem der vorhergehenden Ansprüche, umfassend
- Urformen der ersten und/oder der zweiten und/oder der dritten Messanordnung ganz oder, und/oder
- Urformen von einer, zwei oder mehreren Elektroden der ersten und/oder der zweiten und/oder der dritten Messanordnung ganz oder teilweise aus elektrisch leitfähigem Kunststoff, insbesondere elektrisch leitfähigem Polypropylen, und/oder
- Urformen von einem, zwei oder mehreren Isolierabschnitten der ersten und/oder der zweiten und/oder der dritten Messanordnung ganz oder teilweise ganz oder teilweise aus nicht elektrisch leitfähigem und/oder isolierendem Kunststoff, und/oder
- Urformen von einer, zwei oder mehreren Elektroden der ersten und/oder der zweiten und/oder der dritten Messanordnung und von einem, zwei oder mehreren Isolierabschnitten der ersten Messanordnung.

17. Verwendung eines Multisensors nach mindestens einem der vorhergehenden Ansprüche zur Messung von mindestens drei Parametern in einem Bioreaktor zur Anwendung in der Zellkultur und/oder der Mikrobiologie.

18. Verfahren zur Messung von mindestens drei Parametern in einem Bioreaktor zur Anwendung in der Zellkultur und/oder der Mikrobiologie, das Verfahren umfassend,
- Bereitstellen eines Multisensors nach mindestens einem der vorhergehenden Ansprüche,
- Durchführen einer Impedanzmessung und/oder einer kapazitiven Messung mit einer ersten der drei Messanordnungen,
- Durchführen von zwei weitere Messungen mit der zweiten und dritten der drei Messanordnungen.
